(19)

**Europäisches Patentamt**

**European Patent Office**

**Office européen des brevets**

(11) **EP 1 332 753 A2**

(12) **DEMANDE DE BREVET EUROPEEN**

(43) Date de publication:
**06.08.2003 Bulletin 2003/32**

(51) Int Cl.7: **A61K 7/48**, A61K 7/06, A61K 7/02

(21) Numéro de dépôt: **03290148.0**

(22) Date de dépôt: **21.01.2003**

(84) Etats contractants désignés:
**AT BE BG CH CY CZ DE DK EE ES FI FR GB GR HU IE IT LI LU MC NL PT SE SI SK TR**
Etats d'extension désignés:
**AL LT LV MK RO**

(30) Priorité: **24.01.2002 FR 0200883**
**25.02.2002 FR 0202357**
**24.01.2002 FR 0200885**
**25.02.2002 FR 0202358**

(71) Demandeur: **L'OREAL**
**75008 Paris (FR)**

(72) Inventeurs:
- **Lennon, Paula**
  **69003 Lyon (FR)**
- **Lorant, Raluca**
  **94320 Thiais (FR)**

(74) Mandataire: **Rasson, Catherine**
**L'OREAL,**
**DIPI,**
**6, rue Bertrand Sincholle**
**92585 Clichy Cedex (FR)**

(54) **Composition cosmétique gélifiée par un polymère semi-cristallin**

(57) L'invention se rapporte à une composition sous forme d'émulsion eau-dans-huile, comportant une phase aqueuse dispersée dans une phase huileuse, contenant au moins un émulsionnant ayant une valeur de HLB allant de 1 à 8, au moins une poudre organique et, dans la phase huileuse, au moins un polymère semi-cristallin à structure organique, solide à température ambiante et ayant une température de fusion inférieure à 70°C, comportant a) un squelette polymérique et b) au moins une chaîne organique latérale cristallisable et/ou une séquence organique cristallisable faisant partie du squelette dudit polymère, ledit polymère ayant une masse moléculaire moyenne en nombre supérieure à 2 000.

Cette composition peut constituer notamment une crème cosmétique, ayant une texture très agréable à l'application sur la peau, ou un stick notamment un stick démaquillant.

EP 1 332 753 A2

## Description

La présente invention se rapporte à une composition se présentant sous forme d'une émulsion eau-dans-huile (E/H), ladite composition contenant un émulsionnant, une poudre organique et un polymère particulier dans la phase huileuse, et à ses utilisations, notamment dans les domaines cosmétique ou dermatologique.

Dans les domaines cosmétique ou dermatologique, il est courant d'utiliser des compositions constituées d'une émulsion eau-dans-huile (E/H) comportant une phase aqueuse dispersée dans une phase huileuse. Ces émulsions peuvent constituer des crèmes, une crème étant, dans les domaines considérés, des produits malléables et déformables, par opposition aux compositions solides. Les émulsions E/H comportent une phase continue huileuse et permettent donc de former à la surface de la peau, un film lipidique qui prévient la perte d'eau transépidermique et protège la peau des agressions extérieures. Ces émulsions sont particulièrement appropriées pour protéger et nourrir la peau, et en particulier pour traiter les peaux sèches. En outre, le film lipidique formé à la surface de la peau peut aussi augmenter la rémanence des filtres solaires. Ces émulsions ont aussi l'avantage de permettre la protection et le transport des actifs hydrophiles sensibles à l'oxydation.

Toutefois, les émulsions E/H présentent l'inconvénient d'apporter lors de l'application sur la peau, un toucher assez gras du fait que la phase huileuse est la phase externe. Ainsi, ces émulsions sont en général utilisées pour les peaux sèches, étant trop grasses pour être utilisées sur les peaux grasses. De plus, les émulsions E/H n'apportent aucune fraîcheur et sont généralement trop riches en huiles pour être utilisées pendant l'été ou dans les pays chauds.

Par ailleurs, les émulsions E/H présentent des problèmes de stabilité, notamment quand la phase aqueuse est en quantité importante. Les gouttes de phase aqueuse ont alors tendance à s'agréger et à former des amas visibles au microscope. Cette agrégation est dommageable pour la stabilité des émulsions ; elle favorise d'une part le crémage ou la sédimentation des systèmes fluides, et d'autre part la coalescence des gouttes, conduisant à l'apparition de domaines d'eau, c'est-à-dire de gouttes de phase aqueuse de dimension supérieure à 50 microns. Il est souvent nécessaire, pour stabiliser ces émulsions, d'utiliser un fort taux d'émulsionnants et/ou d'introduire une certaine quantité de facteurs de consistance, tels que les cires. Toutefois, ces facteurs de consistance contribuent à accentuer les défauts cosmétiques (effet poisseux et gras) des émulsions E/H, et il en résulte l'obtention de compositions souvent lourdes. Par ailleurs, en présence de ces facteurs de consistance, il est difficile d'obtenir des émulsions fluides car ces facteurs épaississent les émulsions. En outre, si on augmente fortement la teneur en émulsionnant dans ces émulsions pour remédier à leur instabilité, les émulsions obtenues peuvent se montrer irritantes vis-à-vis de certains types de peau, notamment les peaux sensibles.

De plus, pour les compositions E/H destinées au démaquillage, la présence d'un taux important de cires diminuent les propriétés des compositions, notamment celles destinées au démaquillage de la peau car la présence de cires rend difficile l'élimination du produit démaquillant quand on l'essuie ou quand on le rince (mauvaise rinçabilité).

Il subsiste donc le besoin d'une émulsion E/H, pouvant se présenter notamment sous forme de crème ou sous forme solide, n'ayant pas les inconvénients rencontrés avec celles connues jusqu'à présent, en particulier d'une émulsion au toucher léger et frais, et présentant une bonne stabilité, même en l'absence des facteurs de consistance habituels de la phase huileuse, notamment même en l'absence de cires, et ce même si l'on cherche à obtenir une composition sous forme solide.

De façon surprenante, la demanderesse a trouvé que l'utilisation de polymères particuliers en association avec une ou plusieurs poudres organiques permettait de structurer, même en l'absence de cire, les phases huileuses des émulsions E/H contenant un émulsionnant de HLB défini, et conduisait à une composition donnant un dépôt non collant et frais.

Par "phase huileuse", au sens de la demande, on entend une phase grasse liquide à température ambiante (25°C) et pression atmosphérique (760 mm de Hg), composée notamment d'un ou plusieurs corps gras liquides à température ambiante, appelés aussi huiles, compatibles entre eux. Cette phase huileuse est macroscopiquement homogène.

De façon plus précise, l'invention a pour objet une composition pour application topique, comportant une phase aqueuse dispersée dans une phase huileuse, caractérisée en ce qu'elle contient au moins une poudre organique et au moins un émulsionnant ayant une valeur de HLB allant de 1 à 8, et en ce que la phase huileuse contient au moins un polymère semi-cristallin solide à température ambiante et ayant une température de fusion inférieure à 70°C, ledit polymère comportant a) un squelette polymérique et b) au moins une chaîne organique latérale cristallisable et/ou une séquence organique cristallisable faisant partie dudit squelette polymérique, ledit polymère ayant une masse moléculaire moyenne en nombre supérieure à 2 000.

On entend dans la présente demande par « température ambiante » une température de 25°C.

La présence de polymères semi-cristallins permet d'obtenir des crèmes ayant des textures très fondantes avec un excellent étalement sur la peau. En effet, les propriétés rhéologiques particulières de ces polymères semi-cristallins permettent une fluidification sous l'effet du cisaillement lors de l'application sur la peau. En plus de cela, ces polymères sont intéressants pour leur capacité à se modifier en fonction de la température de la peau. En effet, le

polymère semi-cristallin change d'état en fonction de la température de la peau et de la température de l'environnement. Lorsque la température est inférieure à la température de fusion du polymère, les chaînes de polymère sont rigides et la texture de la composition le contenant est plus compacte et analogue à un baume. Lorsque la température augmente, les chaînes de polymère se relaxent et la texture de la composition devient plus souple. La texture est donc fonction de la température à l'application, et ainsi, la crème qui apparaît compacte dans le pot s'avère en fait très souple lors de l'application sur la peau.

En outre, la composition selon l'invention, du fait de l'association des polymères semi-cristallins et des poudres organiques, présente l'avantage d'avoir une texture agréable, avec un toucher soyeux et un effet satiné mais non-brillant en final. En plus, elle permet d'homogénéiser le teint et de protéger la peau des agressions (froid, vent, chaleur). Ainsi, après application sur la peau, la crème selon l'invention constitue une protection contre les agressions et s'adapte au climat.

Par ailleurs, quand la composition selon l'invention est sous forme solide, elle a tout à la fois les qualités voulues de rigidité pour que la composition solide ne casse pas et les propriétés de transfert suffisantes pour que le dépôt de produit sur la peau se fasse bien et, dans le cas de compositions démaquillantes, permette un bon démaquillage et/ou nettoyage de la peau.

La composition selon l'invention étant destinée à une application topique, notamment sur la peau, les lèvres ou les phanères, elle comprend un milieu physiologiquement acceptable. On entend ici par « milieu physiologiquement acceptable », un milieu non toxique et susceptible d'être appliqué sur la peau (y compris l'intérieur des paupières), les lèvres, les ongles ou les cheveux d'êtres humains. La composition de l'invention peut constituer notamment une composition cosmétique ou dermatologique.

La composition selon l'invention peut notamment constituer une crème ou un onguent, c'est-à-dire un produit souple par opposition à un produit solide tel qu'un stick. Une crème a une viscosité à la température ambiante (environ 25°C) allant d'environ 2 à 250 poises, soit 0,2 à 25 Pa.s, de préférence d'environ 5 à 240 poises, soit 0,5 à 24 Pa.s, et mieux 50 à 200 poises, soit 5 à 20 Pa.s, cette viscosité étant mesurée avec un Rhéomat 180.

Selon une autre forme de réalisation, la composition de l'invention peut être solide et constituer notamment un stick, et en particulier un stick démaquillant. On entend par « composition solide » au sens de la présente invention, toute composition ne s'écoulant pas sous son propre poids, présentant une dureté telle que définie ci-dessous (force de cisaillement de 100 à 350 gf).

Polymères semi-cristallins

Par "polymère semi-cristallin", on entend au sens de l'invention, des polymères comportant une partie cristallisable, chaîne pendante ou séquence dans le squelette, et une partie amorphe dans le squelette, et présentant une température de changement de phase réversible du premier ordre, en particulier de fusion (transition solide-liquide). Par "polymères", on entend au sens de l'invention, des composés comportant au moins 2 motifs répétitifs, de préférence au moins 3 motifs répétitifs et plus spécialement au moins 10 motifs répétitifs. Lorsque la partie cristallisable est une séquence du squelette polymérique, cette séquence cristallisable est de nature chimique différente de celle des séquences amorphes ; le polymère semi-cristallin est dans ce cas un polymère séquencé par exemple du type dibloc, tribloc ou multibloc.

De façon avantageuse, le ou les polymères semi-cristallins de la composition de l'invention ont une masse moléculaire moyenne en nombre Mn supérieure ou égale à 2000, allant par exemple de 2 000 à 800 000, de préférence de 3 000 à 500 000, par exemple de 4 000 à 150 000, et mieux de 4 000 à 99 000.

Dans la composition selon l'invention, les polymères semi-cristallins sont avantageusement solubles dans la phase huileuse à au moins 1 % en poids, à une température supérieure à leur température de fusion. En dehors des chaînes ou séquences cristallisables, les séquences des polymères sont amorphes. Par "chaîne ou séquence cristallisable", on entend au sens de l'invention une chaîne ou séquence qui, si elle était seule, passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitifs du polymère.

De préférence, le squelette polymérique des polymères semi-cristallins est soluble dans la phase huileuse.

De façon préférée, les polymères semi-cristallin utilisés dans la composition de l'invention présentent une température de fusion (ou point de fusion), pF, inférieure à 70°C (25°C ≤pF< 70°C), et mieux inférieure ou égale à 60°C, allant par exemple de 30°C à 60°C (30°C ≤ pF ≤ 60°C), cette température étant au moins égale à la température du support kératinique devant recevoir la composition selon l'invention. Lorsque la composition constitue un produit non solide et en particulier une crème, la température de fusion est de préférence inférieure à 60°C, et mieux inférieure à 50°C (30°C ≤ pF < 50°C). La température de fusion peut être mesurée notamment par toute méthode connue et en particulier avec un calorimètre à balayage différentiel (D.S.C).

De préférence, les séquences ou chaînes cristallisables des polymères semi-cristallins représentent au moins 30 % du poids total de chaque polymère et mieux au moins 40 %. Les polymères semi-cristallins à séquences cristallisables utilisés selon l'invention sont des polymères séquencés ou multiséquencés. Ils peuvent être obtenus par polymérisation de monomère à doubles liaisons réactives (ou éthyléniques) ou par polycondensation. Lorsque les polymères de l'invention sont des polymères à chaînes latérales cristallisables, ils sont avantageusement sous forme aléatoire ou statistique.

De préférence, les polymères semi-cristallins de l'invention sont d'origine synthétique. En outre, ils ne comportent pas de squelette polysaccharidique.

Les polymères semi-cristallins utilisables dans l'invention peuvent être choisis en particulier parmi :

1. les copolymères séquencés de polyoléfines à cristallisation contrôlée, dont les monomères sont décrits dans le document EP-A-951897 ;
2. les polycondensats et notamment les polycondensats polyesters, aliphatiques ou aromatiques, et les copolyesters aliphatiques/aromatiques ;
3. les polymères (homo- ou co-polymères) portant au moins une chaîne latérale cristallisable, et les polymères (homo- ou co-polymères) portant dans le squelette au moins une séquence cristallisable, comme ceux décrits dans le document US-A-5,156,911 ;
4. les polymères (homo- ou co-polymères) portant au moins une chaîne latérale cristallisable à groupement(s) fluoré(s), tels que décrits dans le document WO-A-01/19333 ;
5. et leurs mélanges.

Dans les deux derniers cas (3 et 4), la ou les chaînes latérales ou séquences cristallisables sont hydrophobes.

Les polymères cristallins à chaînes latérales cristallisables ou portant dans le squelette au moins une séquence cristallisable (cas 3 indiqué ci-dessus) sont décrits plus particulièrement ci-dessous.

*A) Polymères semi-cristallins à chaînes latérales cristallisables*

On peut citer en particulier ceux décrits dans les documents US-A-5,156,911 et WO-A-01/19333. Ce sont des homopolymères ou copolymères comportant de 50 à 100 % en poids de motifs résultant de la polymérisation d'un ou de plusieurs monomères porteurs de chaîne(s) latérale(s) hydrophobe(s) cristallisable(s). Ces polymères sont de toute nature du moment qu'ils présentent les conditions indiquées ci-après avec, en particulier, la caractéristique d'être solubles ou dispersables dans la phase huileuse, par chauffage au-dessus de leur température de fusion pF. Ils peuvent résulter :

- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique ;
- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

a) D'une façon générale les motifs (chaînes ou séquences) cristallisables des polymères semi-cristallins selon l'invention proviennent de monomère(s) à séquence(s) ou chaîne(s) cristallisable(s), utilisé(s) pour la fabrication des polymères semi-cristallins. Ces polymères sont choisis notamment parmi les homopolymères et copolymères résultant de la polymérisation d'au moins un monomère à chaîne(s) cristallisable(s) qui peut être représenté par la formule X :

$$\begin{array}{c} -\mathrm{M}- \\ | \\ \mathrm{S} \\ | \\ \mathrm{C} \end{array}$$

où M représente un atome du squelette polymérique, S représente un espaceur et C représente un groupe cristallisable.

Les chaînes « -S-C » cristallisables peuvent être aliphatiques ou aromatiques, éventuellement fluorées ou perfluorées. « S » représente notamment un groupe $(CH_2)_n$ ou $(CH_2CH_2O)_n$ ou $(CH_2O)$, linéaire ou ramifié ou cyclique,

n étant un nombre entier allant de 0 à 22. De préférence « S » est un groupe linéaire. De préférence, « S » et « C » sont différents.

Lorsque les chaînes cristallisables sont des chaînes aliphatiques (alkyle), elles comportent au moins 11 atomes de carbone et au plus 40 atomes de carbone et mieux au plus 24 atomes de carbone. Il s'agit notamment de chaînes alkyle possédant au moins 12 atomes de carbone, et de préférence, il s'agit de chaînes alkyle comportant de 14 à 24 atomes de carbone ($C_{14}$-$C_{24}$). Il peut s'agir de chaînes alkyle hydrocarbonées (atomes de carbone et d'hydrogène) ou chaînes alkyle fluorées ou perfluorées (atomes de carbone, atomes de fluor et éventuellement atomes d'hydrogène). Lorsqu'il s'agit de chaînes alkyle fluorées ou perfluorées, elles comportent au moins 11 atomes de carbone dont au moins 6 atomes de carbone sont fluorés. Les monomères ayant une chaîne alkyle comportant au moins 14 atomes de carbone assurent aux polymères résultant, une structuration de la phase grasse par cristallisation, ce qui n'est pas le cas avec des chaînes alkyle carbonées comportant moins de 14 atomes de carbone.

Comme exemples de polymères ou copolymères semi-cristallins à chaîne(s) cristallisable(s), on peut citer ceux résultant de la polymérisation d'au moins un monomère à chaîne cristallisable choisi parmi les (méth)acrylates d'alkyle saturés en $C_{14}$-$C_{24}$ ($C_{14}$-$C_{24}$ signifie que le groupe alkyle comporte de 14 à 24 atomes de carbone) ; les (méth) acrylates de perfluoroalkyle en $C_{11}$-$C_{15}$ (groupe alkyle avec 11 à 15 atomes de carbone) ; les N-alkyl (méth)acrylamides en $C_{14}$ à $C_{24}$ avec ou sans atome de fluor (groupe alkyle avec 14 à 24 atomes de carbone) ; les esters vinyliques à chaînes alkyle ou perfluoroalkyle en $C_{14}$ à $C_{24}$ (groupe alkyle avec 14 à 24 atomes de carbone), une chaîne perfluoroalkyle comportant au moins 6 atomes de fluor ; les éthers vinyliques à chaînes alkyle ou perfluoroalkyle en $C_{14}$ à $C_{24}$ (groupe alkyle avec 14 à 24 atomes de carbone), une chaîne perfluoroalkyle comportant au moins 6 atomes de fluor ; les alpha-oléfines en $C_{14}$ à $C_{24}$ (groupe alkyle avec 14 à 24 atomes de carbone), comme par exemple l'octadécène ; les para-alkyl styrènes en $C_{14}$ à $C_{24}$ (groupe alkyle avec 14 à 24 atomes de carbone), leurs mélanges.

Par "alkyle", on entend au sens de l'invention un groupement saturé, comportant notamment de 8 à 24 atomes de carbone ($C_8$ à $C_{24}$), sauf mention exprès, et mieux de 14 à 24 atomes de carbone ($C_{14}$ à $C_{24}$).

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères utilisés dans la composition de l'invention sont des copolymères, ils contiennent, en plus, de 0 à 50 % de groupes Y ou Z résultant de la copolymérisation :

$\alpha$) avec Y qui est un monomère polaire ou non polaire ou un mélange des deux :

- Lorsque Y est un monomère polaire, c'est soit un monomère porteur de groupes polyoxyalkylénés (notamment oxyéthylénés et/ou oxypropylénés), un (méth)acrylate d'hydroxyalkyle comme l'acrylate d'hydroxyéthyle, le (méth)acrylamide, un N-alkyl(méth)acrylamide, un NN-dialkyl(méth)acrylamide comme par exemple le NN-diisopropylacrylamide ou la N-vinyl-pyrrolidone (NVP), le N-vinyl-caprolactame, soit un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.

- Lorsque Y est un monomère non polaire, il peut être un ester du type (méth)acrylate d'alkyle linéaire, ramifié ou cyclique, un ester vinylique, un alkyl vinyl éther, une alpha-oléfine, le styrène ou le styrène substitué par un groupe alkyle comportant de 1 à 10 atomes de carbone ($C_1$ à $C_{10}$), comme l'$\alpha$-méthylstyrène, un macromonomère du type polyorganosiloxane à insaturation vinylique.

$\beta$) avec Z qui est un monomère polaire ou un mélange de monomères polaires, Z ayant la même définition que le "Y polaire" défini ci-dessus.

De préférence, les polymères semi-cristallins à chaîne latérale cristallisable sont choisis parmi les homopolymères d'alkyl(méth)acrylate ou d'alkyl(méth)acrylamide avec un groupe alkyle tel que défini ci-dessus, et notamment en $C_{14}$-$C_{24}$ ; les copolymères de ces monomères avec un monomère hydrophile de préférence de nature différente de l'acide (méth)acrylique ; et leurs mélanges. Il peut s'agir par exemple comme copolymères, des copolymères d'alkyl (méth)acrylate ou d'alkyl (méth)acrylamide avec un groupe alkyle en $C_{14}$ à $C_{24}$, avec la N-vinylpyrrolidone, l'hydroxyéthyl (méth)acrylate, l'acide acrylique ; ou leurs mélanges.

*B) Les polymères portant dans le squelette au moins une séquence cristallisable :*

Il s'agit encore de polymères solubles ou dispersables dans la phase huileuse par chauffage au-dessus de leur point de fusion pF. Ces polymères sont notamment des copolymères séquencés constitués d'au moins 2 séquences

de nature chimique différente dont l'une est cristallisable.

On peut utiliser en particulier :

1) Les polymères définis dans le document US-A-5,156,911 ;

2) Les copolymères séquencés d'oléfine ou de cyclooléfine à chaîne cristallisable comme ceux issus de la polymérisation séquencée de :

- cyclobutène, cyclohexène, cyclooctène, norbornène (c'est-à-dire bicyclo(2,2,1)heptène-2), 5-méthylnorbornène, 5-éthylnorbornène, 5,6-diméthyl-norbornène, 5,5,6-triméthyl norbornène, 5-éthylidène-norbornène, 5-phényl-norbonène, 5-benzylnorbornène, 5-vinylnorbornène, 1,4,5,8-diméthano-1,2,3,4,4a,5,8a-octahydronaphtalène, dicyclopentadiène, ou leurs mélanges ;
- avec l'éthylène, le propylène, le 1-butène, le 3-méthyl-1-butène, le 1-hexène, le 4-méthyl-1-pentène, le 1-octène, le 1-décène, le 1-éicosène ou leurs mélanges.

Ces copolymères séquencés peuvent être en particulier les copolymères (éthylène/norbornène) blocs et les terpolymères blocs (éthylène/propylène/éthylidène-norbornène).

On peut aussi utiliser ceux résultant de la copolymérisation séquencée d'au moins 2 $\alpha$-oléfines en $C_2$-$C_{16}$ et mieux en $C_2$-$C_{12}$, tels que ceux cités précédemment et en particulier les bipolymères séquencés d'éthylène et d'1-octène.

3) Les copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :

. Séquence cristallisable par nature : a) polyester comme les poly(alkylène téréphtalate), b) polyoléfine comme les polyéthylènes ou polypropylènes.

. Séquence amorphe et lipophile comme les polyoléfines ou copoly(oléfine)s amorphes telles que le poly(isobutylène), le polybutadiène hydrogéné, le poly(isoprène) hydrogéné.

Comme exemples de tels copolymères à séquence cristallisable et à séquence amorphe, on peut citer :

$\alpha$) les copolymères séquencés poly($\varepsilon$-caprolactone)-b-poly(butadiène), utilisés de préférence hydrogénés, tels que ceux décrits dans l'article "Melting behavior of poly(-caprolactone)-block-polybutadiène copolymers" de S. Nojima, Macromolécules, 32, 3727-3734 (1999).

$\beta$) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).

$\gamma$) les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993), et "Polymer agregates with crystalline cores : the system poly(ethylene)-poly(ethylene-propylene)" P. Richter et al., Macromolécules, 30, 1053-1068 (1997).

$\delta$) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins de la composition de l'invention peuvent être non réticulés ou réticulés en partie, du moment que le taux de réticulation ne gène pas leur dissolution ou dispersion dans la phase huileuse par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère, comme par exemple les interactions dipolaires entre ionomères carboxylates, ces interactions étant en faible quantité et portées par le squelette du polymère ; soit à une séparation de phase entre les séquences cristallisables et les séquences amorphes portées par le polymère.

De préférence, les polymères semi-cristallins de la composition selon l'invention sont non réticulés.

Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en $C_{14}$-$C_{24}$, les (méth)acrylates de perfluoroalkyle en $C_{11}$-$C_{15}$, les N alkyl (méth)acrylamides en $C_{14}$ à $C_{24}$ avec ou

sans atome de fluor, les esters vinyliques à chaînes alkyle ou perfluoroalkyle en $C_{14}$ à $C_{24}$, les éthers vinyliques à chaînes alkyle ou perfluoroalkyle en $C_{14}$ à $C_{24}$, les alpha-oléfines en $C_{14}$ à $C_{24}$, les para-alkyl styrènes avec un groupe alkyle comportant de 12 à 24 atomes de carbone, avec au moins un ester ou amide d'acide monocarboxylique en $C_1$ à $C_{10}$ éventuellement fluoré, qui peut être représenté par la formule suivante :

$$CH_2 = \underset{\displaystyle R_1}{\underset{|}{C}} - \underset{\displaystyle O}{\underset{||}{C}} - X - R$$

dans laquelle $R_1$ est H ou $CH_3$, R représente un groupe alkyle en $C_1$-$C_{10}$ éventuellement fluoré et X représente O, NH ou $NR_2$, où $R_2$ représente un groupe alkyle en $C_1$-$C_{10}$ éventuellement fluoré.

Selon un mode plus particulier de réalisation de l'invention, le polymère est issu d'un monomère à chaîne cristallisable choisi parmi les (méth)acrylates d'alkyle saturés en $C_{14}$-$C_{22}$.

A titre d'exemple particulier de polymère semi-cristallin utilisable dans la composition selon l'invention, on peut citer les produits Intelimer® de la société Landec décrits dans la brochure "Intelimer® polymers". Ces polymères sont sous forme solide à température ambiante (25°C). Ils sont porteurs de chaînes latérales cristallisables et présentent la formule X précédente. On peut citer notamment le « Landec IP22 », ayant une température de fusion pF de 56°C, qui est un produit visqueux à température ambiante, imperméable, non-collant.

On peut aussi utiliser les polymères semi-cristallins décrits dans les exemples 3, 4, 5, 7, 9 du document US-A-5,156,911, résultant de la copolymérisation d'acide acrylique et d'alkyl(méth)acrylate en $C_5$ à $C_{16}$ ayant un pF allant de 20°C à 35°C, et plus particulièrement ceux résultant de la copolymérisation :

- d'acide acrylique, d'hexadécylacrylate et d'isodécylacrylate dans un rapport 1/16/3 ;
- d'acide acrylique et de pentadécylacrylate dans un rapport 1/19 ;
- d'acide acrylique, d'hexadécylacrylate, éthylacrylate dans un rapport 2,5/76,5/20 ;
- d'acide acrylique, d'hexadécylacrylate et de méthylacrylate dans un rapport 5/85/10 ;
- d'acide acrylique, de polyoctadécylméthacrylate dans un rapport 2,5/97,5.

On peut aussi utiliser le polymère « Structure O » commercialisé par la société National Starch, tel que celui décrit dans le document US-A-5,736,125, de pF 44°C, ainsi que les polymères semi-cristallins à chaînes pendantes cristallisables comportant des groupements fluorés tels que décrits dans les exemples 1, 4, 6, 7 et 8 du document WO-A-01/19333.

On peut encore utiliser les polymères semi-cristallins obtenus par copolymérisation d'acrylate de stéaryle et d'acide acrylique ou de NVP, tels que décrits dans le document US-A-5,519,063 ou EP-A-0550745, et plus spécialement ceux décrits dans les exemples 1 et 2, ci-après, de préparation de polymère, de température de fusion respectivement de 40°C et 38°C.

On peut aussi utiliser les polymères semi-cristallins obtenus par copolymérisation de l'acrylate de béhényle et de l'acide acrylique ou de NVP, tels que décrits dans les documents US-A-5,519,063 et EP-A-0550745, et plus spécialement ceux décrits dans les exemples 3 et 4, ci-après, de préparation de polymère, de température de fusion respectivement de 60°C et 58°C.

Selon un mode particulier de réalisation de l'invention, les polymères semi-cristallins utilisés ne comportent pas de groupement carboxylique.

Le ou les polymères utilisés et la quantité de ces polymères sont choisis selon la finalité de la composition désirée et en fonction de l'application particulière envisagée. De manière générale, la composition de l'invention comprend au moins un polymère semi-cristallin choisi parmi les polymères semi-cristallins ayant une température de fusion allant de 50 à 70°C, les polymères semi-cristallins ayant une température de fusion allant de 30 à 50°C, et leurs mélanges.

Quand la composition de l'invention se présente sous forme d'une crème, c'est-à-dire un produit souple par opposition à un produit solide, le polymère utilisé peut être notamment choisi parmi ceux décrits dans les exemples 1 et 2 des documents US-A-5,519,063 ou EP-A-0550745, ayant une température de fusion respectivement de 40°C et 38°C.

Quand la composition est solide, selon un mode préféré de réalisation de l'invention, la composition comprend au moins un polymère semi-cristallin ayant un pF allant de 50 à 70°C. Selon un mode plus particulier de réalisation de l'invention, une composition solide comprend au moins 2 polymères semi-cristallins, c'est-à-dire au moins un polymère semi-cristallin ayant un pF allant de 50° à 70°C et au moins un polymère semi-cristallin ayant un pF allant de

25°C à 50°C et mieux de 30° à 50°C. La quantité totale de polymère semi-cristallin est alors choisie selon la dureté de la composition désirée et en fonction de l'application particulière envisagée. Cette dureté est telle que la composition est autoportée, c'est-à-dire qu'elle se supporte elle-même en restant sous sa forme solide (par exemple bâton) et elle ne s'affaisse pas sous son poids comme le font les crèmes ou les liquides, et qu'elle puisse se déliter aisément pour former un dépôt satisfaisant sur la peau et les lèvres. La dureté des sticks obtenus est mesurée à 20°C au moyen d'un dynanomètre DFGHS 2 de la société INDELCO-CHATILLON se déplaçant à une vitesse de 100 mm/minute. Cette dureté est exprimée comme la force de cisaillement (exprimée en gramme force, gf) nécessaire pour couper un stick de 12,7 mm de diamètre dans ces conditions. Dans la présente demande, la force de cisaillement de la composition va de préférence de 100 à 350 gf, mieux de 120 à 250 gf, et encore mieux de 150 à 220 gf.

En pratique, la quantité totale de polymère semi-cristallin représente, en poids de matière active, de 0,1 à 50% en poids de par rapport au poids total de la composition. Quand la composition est une crème, cette quantité va de préférence 0,5 à 20 % en poids et mieux de 1 à 10 % en poids par rapport au poids total de la composition. Quand la composition est un solide, cette quantité va de préférence 1 à 40 % en poids, et mieux de 5 à 20 % en poids par rapport au poids total de la composition, cette quantité étant de préférence d'au moins de 5 % en poids par rapport au poids total de la composition. Dans le cas d'un stick, de façon avantageuse, le rapport pondéral de polymère semi-cristallin par rapport à la phase huileuse est de 0,20 à 0,60 et mieux de 0,25 à 0,50, pour obtenir un stick dur qui se délite au contact de la peau ou des lèvres et en particulier de dureté allant de 100 à 350 gf.

Phase huileuse

La composition étant sous forme d'émulsion E/H, la phase huileuse gélifiée par le polymère semi-cristallin constitue la phase continue de l'émulsion. Cette phase huileuse peut être présente en une quantité allant par exemple de 10 à 95 % en poids, de préférence de 10 à 80 % en poids, mieux de 15 à 70 % en poids et encore mieux de 20 à 60 % en poids par rapport au poids total de la composition.

Cette phase huileuse contient au moins une huile, notamment une huile cosmétique, et elle peut contenir plusieurs huiles et éventuellement un ou plusieurs autres corps gras.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules $R^1COOR^2$ et $R^1OR^2$ dans laquelle $R^1$ représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam® ;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles telles que les cyclopolydiméthylsiloxanes (cyclométhicones) comme la cyclohexasiloxane (ou cyclohexamethicone) et le cyclopentadiméthylsiloxane (ou cyclopentamethicone) et leurs mélanges ;
  ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; - leurs mélanges.

On entend par « huile hydrocarbonée » dans la liste d'huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires ; les gommes telles que les gommes de silicone (diméthiconol) ; les résines de silicone telles que la trifluorométhyl-C1-4-alkyldimethicone et la trifluoropropyldimethicone ; les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries ; ainsi que les élastomères de silicone comportant une ou plusieurs chaînes oxyalkylénés et notamment oxyéthylénés, tels que le produit commercialisé sous la dénomination « KSG 21 » par la société Shin-Etsu ; et leurs mélanges.

Ces corps gras peuvent être choisis de manière variée par l'homme du métier afin de préparer une composition ayant les propriétés, par exemple de consistance ou de texture, souhaitées.

Quand la composition selon l'invention est destinée au démaquillage de la peau, la phase huileuse de la composition selon l'invention contient au moins une huile démaquillante, et notamment une huile choisie parmi les esters d'acide gras comportant au moins 12 atomes de carbone. L'huile démaquillante peut être notamment choisie dans le groupe comprenant le palmitate d'éthyl-2 hexyle (ou palmitate d'octyle), le myristate d'éthyl-2 hexyle (ou myristate d'octyle), le palmitate d'isopropyle, le myristate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, et leurs mélanges. La quantité d'huile(s) démaquillante(s) peut constituer toute l'huile de la phase huileuse ou une partie. Elle constitue de préférence au moins 10 % en poids par rapport au poids total de la composition. Cette quantité peut aller par exemple de 10 à 95 %, de préférence de 10 à 80 % en poids et mieux de 10 à 60 % en poids par rapport au poids total de la composition.

Par ailleurs, dans les compositions solides, on peut notamment incorporer une cire. Les cires classiques, au sens de la demande, sont celles généralement utilisées dans les domaines cosmétique et dermatologique ; elles sont notamment d'origine naturelle comme la cire d'abeilles, de Candellila, d'Ouricoury, du Japon, de fibres de liège ou de canne à sucre, les cires de paraffine, de lignite, les cires microcristallines de point de fusion > 50°C, la cire de lanoline, la cire de Montan, les ozokérites, les huiles hydrogénées comme l'huile de jojoba hydrogénée (nom CTFA : hydrogenated jojoba oil), mais aussi les cires d'origine synthétique comme les cires de polyéthylène issues de la polymérisation de l'éthylène, les cires obtenues par synthèse de Fischer-Tropsch à point de fusion > 50°C, les esters d'acides gras et les glycérides concrets à 50°C, les cires de silicone comme les alkyle, alcoxy et/ou esters de poly(di)méthylsiloxane solide à 50°C.

Selon un mode particulier de réalisation de l'invention, quand la composition est sous forme solide, elle contient de préférence au moins une cire choisie parmi les cires de polyéthylène, les cires de jojoba hydrogénées et leurs mélanges. On peut utiliser par exemple comme cire, une cire de polyéthylène telle que celle commercialisée sous la dénomination PERFOMALENE 655 par la société New Phase Technologies, qui a un point de fusion de 93°C, et l'huile de jojoba hydrogénée commercialisée par Desert Whale, qui a un point de fusion de 70°C.

<u>Emulsionnant</u>

L'émulsionnant de la composition selon l'invention a une valeur de HLB (hydrophile-lipophile balance) allant de 1 à 8.

L'émulsionnant peut être choisi par exemple parmi les émulsionnants siliconés, les alkylpolyglycosides (APG), les émulsionnants non ioniques dérivés d'acide gras et de polyol, les polyoléfines à terminaison succinique, et leurs mélanges.

Comme émulsionnants siliconés pouvant entrer dans la composition selon l'invention, on peut citer par exemple les dimethicone copolyols et les alkyl diméthicone copolyols. Comme dimethicone copolyol, on peut citer par exemple le mélange de dimethicone copolyol et de dimethicone (polydiméthylsiloxane) (10/90) commercialisé par la société Dow Corning sous la dénomination DC3225C. Selon un mode préféré de réalisation de l'invention, on utilise comme émulsionnant siliconé un alkyl diméthicone copolyol ayant un radical alkyle comportant de 10 à 22 atomes de carbone, tel que le cétyl diméthicone copolyol comme le produit commercialisé sous la dénomination Abil EM-90 par la société Goldschmidt et comme le mélange de cétyl diméthicone copolyol, d'isostearate de polyglycérole (4 moles) et de laurate d'hexyle vendu sous la dénomination ABIL WE 09 par la société Goldschmidt ; le lauryl diméthicone copolyol et par

exemple le mélange d'environ 91 % de lauryl diméthicone copolyol et d'environ 9 % d'alcool isostéarylique, commercialisé sous la dénomination Q2-5200 par la société Dow Corning, et leurs mélanges.

Comme émulsionnants non ioniques dérivés d'acide gras et de polyol, on peut utiliser notamment les esters d'acide gras et de polyol, l'acide gras ayant notamment une chaîne alkyle en C8-C24, et les polyols étant par exemple le glycérol et le sorbitan. Comme esters d'acide gras et de polyol, on peut citer notamment les esters d'acide isostéarique et de polyols, les esters d'acide stéarique et de polyols, et leurs mélanges, en particulier les esters d'acide isostéarique et de glycérol et/ou de sorbitan, par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, le mélange d'isostéarate de sorbitan et d'isostéarate de polyglycérol (3 moles) commercialisé sous la dénomination Arlacel 1690 par la société Unigema. et leurs mélanges.

L'émulsionnant peut être choisi aussi parmi les alkylpolyglycosides ayant un HLB inférieur à 7, par exemple ceux représentés par la formule générale (I) suivante :

$$R\text{-}O\text{-}(G)_x \quad (I)$$

dans laquelle R représente un radical alkyle ramifié et/ou insaturé, comportant de 14 à 24 atomes de carbone, G représente un sucre réduit comportant de 5 à 6 atomes de carbone, et x désigne une valeur allant de 1 à 10 et de préférence de 1 à 4, et G désigne notamment le glucose, le fructose ou le galactose.

Le radical alkyle insaturé peut comprendre une ou plusieurs insaturations éthyléniques, et en particulier une ou deux insaturations éthyléniques.

Comme alkylpolyglycosides de ce type, on peut citer les alkylpolyglucosides (G=glucose dans la formule (I)), et notamment les composés de formule (I) dans laquelle R représente plus particulièrement un radical oléyle (radical insaturé en C18) ou isostéaryle (radical saturé en C18), G désigne le glucose, x est une valeur allant de 1 à 2, notamment l'isostéaryl-glucoside, l'oléyl-glucoside et leurs mélanges. Cet alkylpolyglucoside peut être utilisé en mélange avec un co-émulsionnant, plus spécialement avec un alcool gras et notamment un alcool gras ayant la même chaîne grasse que celle de l'alkylpolyglucoside, c'est-à-dire comportant de 14 à 24 atomes de carbone et ayant une chaîne ramifiée et/ou insaturée, et par exemple l'alcool isostéarylique quand l'alkylpolyglucoside est l'isostéaryl-glucoside, et l'alcool oléylique quand l'alkylpolyglucoside est l'oleylglucoside, éventuellement sous forme d'une composition autoémulsionnante, comme décrit par exemple dans le document WO-A-92/06778. On peut utiliser par exemple le mélange d'isostéaryl-glucoside et d'alcool isostéarylique, commercialisé sous la dénomination Montanov WO 18 par la société SEPPIC.

Comme polyoléfines à terminaison succinique, on peut citer notamment les polyisobutylènes à terminaison succinique estérifiée et leurs sels, notamment les sels de diéthanolamine, tels que les produits commercialisés sous les dénominations Lubrizol 2724, Lubrizol 2722 et Lubrizol 5603 par la société Lubrizol.

L'émulsionnant est de préférence utilisé en une quantité en matière active allant par exemple de 0,1 à 20 % en poids, de préférence de 0,5 à 10 % en poids et mieux de 0,5 à 5 % en poids par rapport au poids total de la composition.

L'émulsionnant est généralement introduit dans la phase huileuse de l'émulsion.

Phase aqueuse

La phase aqueuse de la composition de l'invention représente généralement de 5 à 90 % en poids et de préférence de 20 à 60 % en poids par rapport au poids total de la composition. Elle peut contenir, outre l'eau, des solvants tels que les alcools primaires comportant de 1 à 6 atomes de carbone comme l'éthanol, ou les polyols comme le butylène glycol, la glycérine, le sorbitol, l'hexylène glycol, le propylène glycol et l'isoprène glycol, ou des sucres comme le glucose, le fructose. Le ou les solvants peuvent être présents en une quantité allant de 0,1 à 30 % en poids par rapport au poids total de la composition.

Poudre organique

On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en

copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 μm et masse volumique 40 kg/$m^3$), 551 DE 20 (granulométrie d'environ 30 μm et masse volumique 65 kg/$m^3$), 551 DE 50 (granulométrie d'environ 40 μm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyllysine commercialisée sous la dénomination AMIHOPE LL-11 par la Société Ajinomoto ; les particules de microdispersion de cire, qui ont de préférence des dimensions moyennes inférieures à 1 μm et notamment allant de 0,02 μm à 1 μm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges. La ou les poudres organiques peuvent être présentes par exemple en une quantité allant de 0,1 à 20 % en poids et de préférence de 0,5 à 10 % en poids par rapport au poids total de la composition.

On peut y ajouter une ou plusieurs charges minérales, comme par exemple la silice ; les oxydes métalliques tels que le dioxyde de titane ou l'oxyde de zinc ; le mica ; et leurs mélanges. La quantité de charge(s) minérale(s) peut aller par exemple de 0,05 à 20 % en poids et mieux 0,1 à 10 % en poids par rapport au poids total de la composition.

Selon un mode préféré de réalisation de l'invention, la ou les poudres organiques sont ajoutées à la composition après mélange des phases aqueuse et huileuse.

## Additifs

La composition de l'invention peut comprendre, en outre, tout additif usuellement utilisé dans le domaine concerné, choisi parmi les épaississants ou gélifiants, les matières colorantes, les antioxydants, les huiles essentielles, les conservateurs, les parfums, les dispersants, les neutralisants, les actifs lipohiles ou hydrophiles, les électrolytes tels que le sulfate de magnésium ou le chlorure de sodium, et leurs mélanges. Ces additifs peuvent être présents dans la composition selon les quantités généralement utilisées dans le domaine cosmétique et dermatologique et notamment à raison de 0,01 à 50% du poids total de la composition et mieux de 0,1 à 20%. L'eau peut représenter jusqu'à 90 % du poids total de la composition.

Comme actifs, on peut citer par exemple les hydratants et par exemple les hydrolysats de protéines et les polyols tels que la glycérine, les glycols comme les polyéthylène glycols, et les dérivés de sucre ; les extraits naturels ; les oligomères procyannidoliques ; les vitamines comme la vitamine E (tocophérol) et ses dérivés (par exemple acétate), la vitamine A (rétinol) et ses dérivés (par exemple palmitate de rétinyle), la vitamine C (acide ascorbique) et ses dérivés (par exemple palmitate d'ascorbyle), les dérivés de ces vitamines étant notamment des esters dont le palmitate et l'acétate ; les acides gras essentiels ; les sphingolipides et céramides ; les composés auto-bronzants tels que la DHA (dihydroxyacétone) ; les filtres solaires comme par exemple l'octylméthoxycinnamate (Parsol MCX), la 3-benzophénone (Uvinul M40), le butylméthoxydibenzoyl-méthane (Parsol 1789) ; l'urée ; les dépigmentants tels que l'acide kojique et l'acide caféique ; les bêta-hydroxyacides tels que l'acide salicylique et ses dérivés ; les alpha-hydroxyacides tels que l'acide lactique, l'acide citrique et l'acide glycolique ; les rétinoïdes tels que le rétinol et ses esters, le rétinal, les caroténoïdes ; et leurs mélanges.

Comme gélifiants, on peut utiliser notamment les gélifiants hydrophiles tels que les polymères carboxyvinyliques, comme les carbomers ; les polyacrylamides et les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Clariant sous la dénomination commerciale « Hostacerin AMPS » (nom CTFA : ammonium polyacryldimethyltauramide) ; ou le copolymère acrylamide /acrylamido-2-methylpropane sulfonate de sodium en émulsion inverse à 40 % dans le polysorbate, commercialisé sous la dénomination SIMULGEL 600 par la société SEPPIC ; les polysaccharides tels que la gomme de xanthane ; et leurs mélanges.

Ces gélifiants, lorsqu'ils sont présents, sont généralement utilisés à des concentrations allant de 0,05 à 7 % et de préférence de 0,1 à 5 % en poids de matière active par rapport au poids total de la composition.

Bien entendu l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

La composition selon l'invention peut être fabriquée par les procédés connus, généralement utilisés dans le domaine cosmétique ou dermatologique. Elle peut être fabriquée par le procédé qui consiste à chauffer le polymère au moins à sa température de fusion (pF), à y ajouter le ou les émulsionnants et autres constituants de la phase huileuse, à préparer la phase aqueuse à une température supérieure au point de fusion (pF) du polymère et à introduire la phase aqueuse dans la phase huileuse sous agitation, puis à ajouter la ou les poudres à chaud ou à froid selon la composition recherchée. Les compositions sous forme de crème sont refroidies telles quelles tandis que les compositions solides sont généralement, avant refroidissement, coulées dans un moule approprié comme un moule de sticks ou directement dans les articles de conditionnement (boîtier ou coupelle notamment).

La composition selon l'invention trouve son application dans un grand nombre de traitements notamment cosmétiques de la peau, y compris du cuir chevelu, des cheveux, des ongles, et/ou des muqueuses. Elle peut être utilisée pour tout type de peaux, et en particulier pour le soin, le traitement, le nettoyage et/ou la protection notamment solaire, de la peau, des cheveux et/ou des lèvres ou des muqueuses, et pour le maquillage de la peau et/ou des lèvres, ainsi que pour la préparation d'une crème destinée au traitement de la peau, plus particulièrement de la peau grasse.

La composition de l'invention peut constituer aussi une composition de maquillage après ajout de pigments et/ou colorants, et elle peut constituer en outre une base de maquillage (crème mise avant le fond de teint ou la poudre) très efficace et permettant une bonne adhérence de la couche de maquillage constituée par le fond de teint ou la poudre.

Aussi, la présente invention a pour objet l'utilisation cosmétique d'une composition cosmétique telle que définie ci-dessus, pour le traitement, la protection, le soin, le démaquillage et/ou le nettoyage de la peau, des lèvres et/ou des cheveux, et/ou pour le maquillage de la peau et/ou des lèvres.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition cosmétique telle que définie ci-dessus.

L'invention a aussi pour objet l'utilisation de la composition telle que définie ci-dessus pour la fabrication d'une crème destinée au traitement des peaux grasses.

La composition de l'invention peut aussi avantageusement constituer une composition de démaquillage ou de nettoyage de la peau, des lèvres et des phanères, notamment pouvant se présenter sous forme solide, par exemple sous forme d'un stick (ou bâton) ou sous forme coulée. Cette composition est apte à démaquiller ou nettoyer la peau, les lèvres ou les phanères des êtres humains.

L'invention a encore pour objet un procédé cosmétique de démaquillage ou de nettoyage de la peau, des lèvres et des phanères, consistant à appliquer sur la peau, les lèvres ou les phanères, de la composition notamment cosmétique solide telle que définie ci-dessus. Cette composition solide peut être plus spécialement un stick, facile à utiliser et permettant un nouveau geste de démaquillage.

L'invention est illustrée plus en détail dans les exemples suivants. Les quantités sont données en pourcentage massique.

**I) Exemples de fabrication de polymères semi-cristallins**

**Exemple 1 : Polymère acide de point de fusion de 40°C**

Dans un réacteur d'1 litre muni d'une agitation centrale avec ancre, d'un réfrigérant et d'un thermomètre, on introduit 120 g d'huile de Parléam® (huile minérale) que l'on chauffe de la température ambiante à 80°C en 45 min. A 80°C, on introduit en 2h le mélange $C_1$ suivant :
40 g de cyclohexane + 4 g de Triganox 141 [ 2,5 bis (2-éthyl hexanoyl peroxy) - 2,5 - diméthyl hexane].
30 min après le début de la coulée du mélange $C_1$, on introduit en 1h30 le mélange $C_2$ constitué de :
190 g d'acrylate de stéaryle + 10 g d'acide acrylique + 400 g de cyclohexane.

A la fin des deux coulées, on laisse agir 3h supplémentaires à 80°C puis on distille à pression atmosphérique la totalité du cyclohexane présent dans le milieu réactionnel.
On obtient alors le polymère à 60 % en poids en matière active dans l'huile de Parléam®.
Sa masse moléculaire moyenne en poids $M_w$ est de 35 000 exprimée en équivalent polystyrène et sa température de fusion pF est de 40°C ± 1°C, mesurée par D.S.C.

**Exemple 2 : Polymère basique de point de fusion de 38°C**

On applique le même mode opératoire que dans l'exemple 1, sauf que l'on utilise de la N-Vinyl Pyrrolidone en lieu et place de l'acide acrylique.

Le polymère obtenu est à 60% en poids en matière active dans l'huile de Parléam®, sa masse moléculaire moyenne en poids $M_w$ est de 38 000 et son pF de 38°C.

**Exemple 3 : Polymère Acide de point de fusion de 60°C**

On applique le même mode opératoire que dans l'exemple 1, sauf que l'on utilise de l'acrylate de béhényle en lieu et place de l'acrylate de stéaryle. Le polymère obtenu est à 60% en poids en matière active dans l'huile de Parléam®. Sa masse moléculaire moyenne en poids $M_w$ est de 42 000 et son pF de 60°C.

**Exemple 4 : Polymère basique de point de fusion de 58°C**

On applique le même mode opératoire que dans l'exemple 2, sauf que l'on utilise de l'acrylate de béhényle en lieu et place de l'acrylate de stéaryle. Le polymère obtenu est à 60% en poids en matière active dans l'huile de Parléam®. Son $M_w$ est de 45 000 et son pF est de 58°C.

**II) Exemples de composition**

**Exemple 5 : crème de soin**

| *Phase huileuse* | |
|---|---|
| Copolymère acrylate de stéaryle/N-vinylpyrrolidone à 60% en matière active dans l'huile de Parléam® selon exemple 2 | 2 % |
| Isohexadécane | 15 % |
| Huile de silicone | 10 % |
| Cétyl diméthicone copolyol (Abil EM90 de la société Goldschmidt) | 1,5 % |
| Isostéarate de Polyglycérole (Isolan GI 34) | 0,5 % |
| *Phase aqueuse* | |
| Glycérine | 3 % |
| Sulfate de magnésium | 0,7 % |
| Conservateurs | qs % |
| Eau déminéralisée | qsp 100 % |
| *Poudre organique* | |
| Poudre d'amidon | 3 % |

Mode opératoire: on chauffe chacune des phases à chaud (>70°C), puis on disperse la phase aqueuse dans la phase huileuse sous agitation. On refroidit à 40°C et on ajoute la poudre d'amidon.

On obtient une crème de soin blanche, soyeuse, douce et glissante à l'application. L'aspect dans le pot est compact mais au toucher, la crème fond pour s'étaler facilement avec une sensation de fraîcheur. La peau devient plus douce, plus soyeuse et mieux hydratée.

**Exemple 6 : crème de soin**

| *Phase huileuse* | |
|---|---|
| Copolymère acrylate de stéaryle/acide acrylique à 60% en matière active dans l'huile de Parléam® selon exemple 1 | 2 % |
| Isohexadécane | 15 % |
| Huile de silicone | 8 % |

(suite)

| Phase huileuse | |
|---|---|
| Huile minérale | 2 % |
| Cétyl diméthicone copolyol (Abil EM90 de la société Goldschmidt) | 1,5 % |
| Isostéarate de Polyglycérole (Isolan GI 34) | 0,5 % |
| Mica-oxyde de titane (Flamenco Red 420 C de la Société Engelhard) | 1 % |
| *Phase aqueuse* | |
| Glycérine | 3 % |
| Sulfate de magnésium | 0,7 % |
| Conservateurs | qs % |
| Eau déminéralisée | qsp 100 % |
| *Poudre organique* | |
| Microsphères chlorure de vinylidene/acrylonitrile/methacrylate (Expancel 551 DE) | 0,5 % |

Mode opératoire : on chauffe chacune des phases à chaud (>70°C), puis on disperse la phase aqueuse dans la phase huileuse sous agitation. On refroidit à 40°C et on ajoute la poudre organique.
On obtient une belle crème nacrée, douce et glissante à l'application. Cette crème s'étale facilement sur la peau en donnant une sensation de fraîcheur et en laissant la peau douce et satinée.

**Exemple 7 : crème de soin**

| *Phase huileuse* | |
|---|---|
| Copolymère acrylate de stéaryle/N-vinylpyrrolidone à 60% en matière active dans l'huile de Parléam® selon exemple 2 | 3 % |
| Isohexadécane | 8 % |
| Huile de silicone volatile | 3 % |
| Perhydrosqualène végétal raffiné Isostéarate de sorbitan/isostéarate de polylgycérol (3 moles) | 5 % |
| (Arlacel 1690) | 4 % |
| Isostéarate de Polyglycérole (Isolan GI 34) | 0,5 % |
| *Phase aqueuse* | |
| Glycérine | 3 % |
| Sulfate de magnésium | 0,7 % |
| Conservateurs | qs % |
| Eau déminéralisée | qsp 100 % |
| *Poudre organique* | |
| Poudre de polyamide (Orgasol 2002 NAT COS) | 3 % |
| Parfum | 0,5 % |

Mode opératoire : on chauffe chacune des phases à chaud (>70°C), puis on disperse la phase aqueuse dans la phase huileuse sous agitation. On refroidit à 40°C et on ajoute la poudre de polyamide et le parfum.
On obtient une belle crème blanche, riche et confortable, convenant en particulier pour les peaux normales à sèches.

**Exemple 8 : Crème de teint**

| *Phase huileuse* | |
|---|---|
| Copolymère acrylate de stéaryle/N-vinylpyrrolidone à 60% en matière active dans l'huile de Parléam® selon exemple 2 | 2 % |
| Isohexadécane | 10 % |
| Huile de silicone volatile | 7 % |
| Isononylnonanoate | 5 % |
| Huile d'amande douce | 0,5 % |
| Mélange d'isostéarylglucoside et d'alcool isostéarique (Montanov WO18 de Seppic) | 5 % |
| Isostéarate de Polyglycérole (Isolan GI34 de Goldschmidt) | 0,5 % |
| Oxydes de fer (brun, jaune et noir) enrobés | 5 % |
| *Phase aqueuse* | |
| Glycérine | 3 % |
| Sulfate de magnésium | 0,7 % |
| Conservateurs | qs % |
| Eau déminéralisée | qsp 100 % |
| *Poudres organiques* | |
| Poudre de polyamide (Orgasol 2002 NAT COS) | 6 % |
| Poudre de polyéthylène | 4 % |
| Parfum | 0,5 % |

Mode opératoire : on chauffe la phase huileuse et la phase aqueuse à environ 70°C, puis on disperse la phase aqueuse dans la phase huileuse sous agitation. On refroidit à 50°C, ensuite on ajoute la poudre organique. On refroidit sous très faible agitation.

On obtient une belle crème colorée, douce et non-grasse au toucher, qui s'étale facilement et qui lisse la peau et homogénéise le teint.

**Exemple 9 : stick démaquillant**

| *Phase huileuse* | |
|---|---|
| Copolymère acrylate de stéaryle/acide acrylique à 60 % en matière active dans l'huile de Parléam selon l'exemple 1 | 12 % |
| Copolymère acrylate de béhényle/acide acrylique à 60% en matière active dans l'huile de Parléam selon l'exemple 3 | 10 % |
| Palmitate d'isopropyle | 45 % |
| Mélange de cétyl diméthicone copolyol, isostearate de polyglycérole (4 moles), laurate d'hexyle (ABIL WE 09 de la société Goldschmidt) | 5 % |
| Acrylate copolymer (EXPANCEL 551 DE 20D60 de la société Expancel) | 0,1 % |
| *Phase aqueuse* | |
| Glycérine | 5 % |
| Sulfate de magnésium | 0,7 % |
| Conservateurs | 0,8 % |

(suite)

| *Phase aqueuse* | |
|---|---|
| Eau déminéralisée | qsp 100 % |

Mode opératoire: on chauffe la phase huileuse à 80°C. On prépare la phase aqueuse à 80°C et on ajoute sous agitation la phase aqueuse dans la phase huileuse.

On obtient un stick homogène, au toucher fondant, qui s'étale bien et qui n'est pas collant. Ce stick assure un bon démaquillage de la peau.

**Exemple 10 : stick démaquillant**

| *Phase huileuse* | |
|---|---|
| Copolymère acrylate de stéaryle/acide acrylique à 60 % en matière active dans l'huile de Parléam selon l'exemple 1 | 12 % |
| Copolymère acrylate de béhényle/acide acrylique à 60% en matière active dans l'huile de Parléam selon exemple 3 | 10 % |
| Isoparaffine | 15 % |
| Palmitate d'isopropyle | 30 % |
| Mélange de cétyl diméthicone copolyol, isostearate de polyglycérole (4 moles), laurate d'hexyle (ABIL WE 09 de la société Goldschmidt) | 5 % |
| Acrylate copolymer (EXPANCEL 551 DE 20D60 de la société Expancel) | 0,8 % |
| *Phase aqueuse* | |
| Glycérine | 5 % |
| Sulfate de magnésium | 0,7 % |
| Conservateurs | 0,8 % |
| Eau déminéralisée | qsp 100 % |

Mode opératoire : on chauffe la phase huileuse à 80°C. On prépare la phase aqueuse à 80°C et on ajoute sous agitation la phase aqueuse dans la phase huileuse.

On obtient un stick homogène au toucher fondant, doux à l'application, glissant, et non collant. Ce stick permet d'obtenir un démaquillage de la peau efficace et doux.

**Exemple 11 : stick démaquillant**

| *Phase huileuse* | |
|---|---|
| Huile de jojoba hydrogénée de la société Desert Whale (Point de fusion 70°C) | 7 % |
| Cire de polyéthylène (PERFOMALENE 655 | 2 % |
| Copolymère acrylate de béhényle/acide acrylique à 60% en matière active dans le Parléam selon exemple 3 | 8 % |
| Mélange de cétyl diméthicone copolyol, isostearate polyglycérole (4 moles), laurate d'hexyle (ABIL WE 09 de la société Goldschmidt) | 5 % |
| Palmitate d'isopropyle | 45 % |
| Nylon-12 (ORGASOL 2002 EXD NAT COS de la société Atochem) | 1,5 % |

(suite)

| *Phase aqueuse* | |
|---|---|
| Glycérine | 5 % |
| Sulfate de magnésium | 0,7 % |
| Conservateurs | 0,8 % |
| Eau déminéralisée | qsp 100 % |

Mode opératoire : on chauffe la phase huileuse à 80°C. On prépare la phase aqueuse à 80°C et on ajoute sous agitation la phase aqueuse dans la phase huileuse.

On obtient un stick homogène de texture riche, doux à l'application et assurant un bon démaquillage de la peau.

**Exemple 12 : stick démaquillant**

| *Phase huileuse* | |
|---|---|
| Huile de jojoba hydrogénée de la société Desert Whale (Point de fusion 70°C) | 7 % |
| Cire de polyéthylène (PERFOMALENE 655 | 2 % |
| Copolymère acrylate de béhényle/acide acrylique à 60% en matière active dans le Parléam selon exemple 3 | 8 % |
| Mélange de cétyl diméthicone copolyol, isostearate polyglycérole (4 moles), laurate d'hexyle (ABIL WE 09 de la société Goldschmidt) | 5 % |
| Palmitate d'isopropyle | 39 % |
| Huile de silicone (DC 200 FLUID - 5 CST de la société Dow Corning) | 5 % |
| Huile d'abricot | 1 % |
| Nylon-12 (ORGASOL 2002 EXD NAT COS de la société Atochem) | 1,5 % |
| *Phase aqueuse* | |
| Glycérine | 5 % |
| Sulfate de magnésium | 0,7 % |
| Conservateurs | 0,8 % |
| Eau déminéralisée | qsp 100 % |

Mode opératoire : on chauffe la phase huileuse à 80°C. On prépare la phase aqueuse à 80°C et on ajoute sous agitation la phase aqueuse dans la phase huileuse.

On obtient un stick homogène ayant une texture riche et fondante, très confortable à l'application et permettant d'obtenir un démaquillage de la peau, doux et efficace.

**Exemple 13 : stick démaquillant Bi-gel**

| *Phase huileuse* | |
|---|---|
| Copolymère acrylate de stéaryle/acide acrylique à 60 % en matière active dans l'huile de Parléam selon l'exemple 1 | 12 % |
| Copolymère acrylate de béhényle/acide acrylique à 60% en matière active dans l'huile de Parléam selon l'exemple 3 | 10 % |
| Palmitate d'isopropyle | 45 % |
| Mélange de cétyl diméthicone copolyol, isostearate polyglycérole (4 moles), laurate d'hexyle (ABIL WE 09 de la société Goldschmidt) | 5 % |

(suite)

| *Phase huileuse* | |
|---|---|
| Nylon-12 (ORGASOL 2002 EXD NAT COS de la société Atochem) | 1,5 % |

| *Phase aqueuse* | |
|---|---|
| Glycérine | 5 % |
| Ammonium polyacryloyldimethyl taurate (HOSTACERIN AMPS) | 0,5 % |
| Conservateurs | 0,8 % |
| Eau déminéralisée | qsp 100 % |

Mode opératoire : on chauffe la phase huileuse à 80°C. On prépare la phase aqueuse à 80°C et on ajoute sous agitation la phase aqueuse dans la phase huileuse.

On obtient un stick homogène de texture fondante et fraîche à la fois, qui assure un bon démaquillage de la peau.

**Exemple 14:** Cet exemple est réalisé en remplaçant dans l'exemple 9, le copolymère de l'exemple 1, par le copolymère de l'exemple 2 à base d'acrylate de stéaryle et de N-vinylpyrrolidone.

**Revendications**

**1.** Composition pour application topique, comportant une phase aqueuse dispersée dans une phase huileuse, **caractérisée en ce qu'**elle contient au moins une poudre organique et au moins un émulsionnant ayant une valeur de HLB allant de 1 à 8, et **en ce que** la phase huileuse contient au moins un polymère semi-cristallin solide à température ambiante et ayant une température de fusion inférieure à 70°C, ledit polymère comportant a) un squelette polymérique et b) au moins une chaîne organique latérale cristallisable et/ou une séquence organique cristallisable faisant partie dudit squelette polymérique, ledit polymère ayant une masse moléculaire moyenne en nombre supérieure à 2 000.

**2.** Composition selon la revendication 1, **caractérisée en ce que** le polymère a une masse moléculaire moyenne en nombre allant de 3 000 à 500 000 et mieux de 4 000 à 99 000.

**3.** Composition selon la revendication 1 ou 2, **caractérisée en ce que** le polymère est soluble dans la phase huileuse à au moins 1 % en poids à une température supérieure à sa température de fusion.

**4.** Composition selon l'une quelconque des revendications précédentes, **caractérisée en ce que** le polymère a une température de fusion pF telle que 30°C ≤ pF ≤ 60°C.

**5.** Composition selon l'une quelconque des revendications précédentes, **caractérisée en ce que** le polymère est choisi parmi :

- les copolymères séquencés de polyoléfines à cristallisation contrôlée,
- les polycondensats polyesters aliphatiques ou aromatiques et les copolyesters aliphatiques/aromatiques,
- les homo- ou co-polymères portant au moins une chaîne latérale cristallisable, et les homo- ou co-polymères portant dans le squelette au moins une séquence cristallisable,
- les homo- ou co-polymères portant au moins une chaîne latérale cristallisable à groupement(s) fluoré(s),
- et leurs mélanges.

**6.** Composition selon l'une quelconque des revendications précédentes, **caractérisée en ce que** le polymère est choisi parmi les homopolymères et copolymères comportant de 50 à 100 % en poids de motifs résultant de la polymérisation d'un ou de plusieurs monomères porteurs de chaîne(s) latérale(s) hydrophobe(s) cristallisable(s).

**7.** Composition selon l'une quelconque des revendications précédentes, **caractérisée en ce que** le polymère est choisi parmi les homopolymères et copolymères résultant de la polymérisation d'au moins un monomère à chaîne (s) cristallisable(s) de formule X :

où M représente un atome du squelette polymérique, S représente un espaceur et C représente un groupe cristallisable.

8. Composition selon l'une quelconque des revendications précédentes, **caractérisée en ce que** le polymère est choisi parmi les homopolymères et copolymères résultant de la polymérisation d'au moins un monomère à chaîne cristallisable choisi parmi les (méth)acrylates d'alkyle saturés en $C_{14}$-$C_{24}$, les (méth)acrylates de perfluoroalkyle en $C_{11}$-$C_{15}$, les N alkyl (méth)acrylamides en $C_{14}$ à $C_{24}$ avec ou sans atome de fluor, les esters vinyliques à chaînes alkyle ou perfluoroalkyle en $C_{14}$ à $C_{24}$, les éthers vinyliques à chaînes alkyle ou perfluoroalkyle en $C_{14}$ à $C_{24}$, les alpha-oléfines en $C_{14}$ à $C_{24}$, les para-alkyl styrènes avec un groupe alkyle comportant de 12 à 24 atomes de carbone, et leurs mélanges.

9. Composition selon l'une quelconque des revendications précédentes, **caractérisée en ce que** le polymère est choisi parmi les copolymères résultant de la polymérisation d'au moins un monomère à chaîne cristallisable choisi parmi les (méth)acrylates d'alkyle saturés en $C_{14}$-$C_{24}$, les (méth)acrylates de perfluoroalkyle en $C_{11}$-$C_{15}$, les N alkyl (méth)acrylamides en $C_{14}$ à $C_{24}$ avec ou sans atome de fluor, les esters vinyliques à chaînes alkyle ou perfluoroalkyle en $C_{14}$ à $C_{24}$, les éthers vinyliques à chaînes alkyle ou perfluoroalkyle en $C_{14}$ à $C_{24}$, les alpha-oléfines en $C_{14}$ à $C_{24}$, les para-alkyl styrènes avec un groupe alkyle comportant de 12 à 24 atomes de carbone, avec au moins une ester ou amide d'acide monocarboxylique en $C_1$ à $C_{10}$ éventuellement fluoré.

10. Composition selon l'une quelconque des revendications précédentes, **caractérisée en ce que** le polymère est choisi parmi les homopolymères d'alkyl(méth)acrylate ou d'alkyl(méth)acrylamide en $C_{14}$ à $C_{24}$ ; des copolymères de ces monomères avec un monomère hydrophile ; et leurs mélanges.

11. Composition selon l'une quelconque des revendications précédentes, **caractérisée en ce que** le polymère est choisi parmi les copolymères d'alkyl(méth)acrylate ou d'alkyl (méth)acrylamide avec un groupe alkyle en $C_{14}$ à $C_{24}$, avec la N-vinylpyrrolidone, l'hydroxyéthyl (méth)acrylate, l'acide acrylique ; ou leurs mélanges.

12. Composition selon l'une quelconque des revendications précédentes, **caractérisée en ce que** le polymère est issu d'un monomère à chaîne cristallisable choisi parmi les (méth)acrylates d'alkyle saturés en $C_{14}$-$C_{22}$.

13. Composition selon l'une quelconque des revendications précédentes, **caractérisée en ce qu'**elle comprend au moins un polymère semi-cristallin choisi parmi les polymères semi-cristallins ayant une température de fusion allant de 50 à 70°C, les polymères semi-cristallins ayant une température de fusion allant de 30 à 50°C, et leurs mélanges.

14. Composition selon l'une quelconque des revendications précédentes, **caractérisée en ce que** le polymère représente de 0,1 à 50 % en poids, et de préférence de 0,5 à 20 % en poids par rapport au poids total de la composition.

15. Composition selon l'une quelconque des revendications précédentes, **caractérisée en ce que** la phase huileuse représente de 10 à 95 % en poids et de préférence de 10 à 80 % en poids par rapport au poids total de la composition.

16. Composition selon l'une quelconque des revendications précédentes, **caractérisée en ce que** la phase huileuse contient au moins une huile choisie parmi les huiles hydrocarbonées d'origine animale, les huiles hydrocarbonées d'origine végétale, les esters et les éthers de synthèse, les hydrocarbures linéaires ou ramifiés, d'origine minérale ou synthétique, les alcools gras ayant de 8 à 26 atomes de carbone, les huiles fluorées partiellement hydrocarbonées et/ou siliconées, les huiles de silicone, et leurs mélanges.

17. Composition selon l'une quelconque des revendications précédentes, **caractérisée en ce que** la phase huileuse

contient au moins une huile choisie parmi les esters d'acide gras comportant au moins 12 atomes de carbone.

**18.** Composition selon l'une quelconque des revendications précédentes, **caractérisée en ce que** l'émulsionnant est choisi parmi les émulsionnants siliconés, les émulsionnants non ioniques dérivés d'acide gras et de polyol, les alkylpolyglycosides, les polyoléfines à terminaison succinique ; et leurs mélanges.

**19.** Composition selon la revendication précédente, **caractérisée en ce que** l'émulsionnant siliconé est choisi parmi les dimethicone copolyols et les alkyl diméthicone copolyols.

**20.** Composition selon l'une quelconque des revendications précédentes, **caractérisée en ce que** l'émulsionnant est choisi parmi les alkylpolyglycosides représentés par la formule générale (I) suivante :

$$R\text{-}O\text{-}(G)_x \qquad (I)$$

dans laquelle R représente un radical alkyle ramifié et/ou insaturé, comportant de 14 à 24 atomes de carbone, G représente un sucre réduit comportant de 5 à 6 atomes de carbone, x désigne une valeur allant de 1 à 10, et G désigne le glucose, le fructose ou le galactose.

**21.** Composition selon l'une quelconque des revendications précédentes, **caractérisée en ce que** l'émulsionnant est choisi parmi les esters d'acide isostéarique et de polyols, les esters d'acide stéarique et de polyols, et leurs mélanges.

**22.** Composition selon l'une quelconque des revendications précédentes, **caractérisée en ce que** l'émulsionnant est présent en une quantité en matière active allant de 0,1 à 20 % en poids et de préférence de 0,5 à 10 % en poids par rapport au poids total de la composition.

**23.** Composition selon l'une quelconque des revendications précédentes, **caractérisée en ce que** la poudre organique est choisie parmi les particules de polyamide ; les poudres de polyéthylène ; les microsphères à base de copolymères acryliques ; les microsphères de polyméthacrylate de méthyle ; les poudres de copolymère éthylène-acrylate ; les poudres expansées ; les poudres d'amidon ; les poudres d'aminoacides ; les particules de microdispersion de cire ; et leurs mélanges.

**24.** Composition selon l'une quelconque des revendications précédentes, **caractérisée en ce que** la poudre organique est présente en une quantité allant de 0,1 à 20 % en poids et de préférence de 0,5 à 10 % en poids par rapport au poids total de la composition.

**25.** Composition selon l'une quelconque des revendications précédentes, **caractérisée en ce qu'**elle constitue une crème.

**26.** Composition selon l'une quelconque des revendications 1 à 24, **caractérisée en ce qu'**elle se présente sous forme solide.

**27.** Composition selon l'une quelconque des revendications précédentes, **caractérisée en ce qu'**elle constitue une composition cosmétique ou dermatologique.

**28.** Utilisation cosmétique d'une composition cosmétique selon l'une quelconque des revendications 1 à 26, pour le traitement, la protection, le soin, le démaquillage et/ou le nettoyage de la peau, des lèvres et/ou des cheveux, et/ou pour le maquillage de la peau et/ou des lèvres.

**29.** Procédé de traitement cosmétique de la peau, y compris du cuir chevelu, des cheveux, et/ou des lèvres, **caractérisé par le fait que** l'on applique sur la peau, les cheveux et/ou les lèvres, une composition cosmétique selon l'une quelconque des revendications 1 à 26.

**30.** Procédé cosmétique de démaquillage ou de nettoyage de la peau, des lèvres et des phanères, consistant à appliquer sur la peau, les lèvres ou les phanères, une composition cosmétique selon la revendication 26.

**31.** Utilisation de la composition selon l'une quelconque des revendications 1 à 25 pour la fabrication d'une crème destinée au traitement des peaux grasses.